# EUROPEAN PATENT APPLICATION

(11) **EP 3 473 178 A1**
(43) Date of publication of application: **24.04.2019**
(21) Application number: 18201611.3
(22) Date of filing: 19.10.2018
(51) Int. Cl.: A61B 5/145, A61B 5/00, A61B 5/11

(54) **MEASURING APPARATUS, MEASURING PROGRAM AND MEASURING METHOD**

(30) Priority: 20.10.2017 JP 2017203624; 16.10.2018 JP 2018194788
(71) Applicant: ARKRAY, Inc., Kyoto-shi, Kyoto 601-8045 (JP)
(72) Inventor: SHIMIZU, Takeshi, Kyoto-shi, Kyoto 602-0008 (JP); KUSAKA, Yasuhide, Kyoto-shi, Kyoto 602-0008 (JP)
(74) Representative: Dehns

(57) **Abstract**

A measuring apparatus (1) includes: a measurement unit (12) configured to measure a concentration value of a specified substance contained in a sample; an acquiring unit (13) configured to acquire information about a user's activity status; a calculation unit (13) configured to calculate a concentration value of the specified substance within a second period ranging from present time to second predetermined time after the present time, based on a concentration value of the specified substance within a first period ranging from first predetermined time before the present time to the present time and the information about the user's activity status within the first period; and an informing unit (13) configured to inform a user of the information about the concentration value of the specified substance when the concentration value of the specified substance within the second period does not fall within a range of a predetermined concentration value.

## Description

### FIELD

The invention pertains to a measuring apparatus, a measuring program and a measuring method.

### BACKGROUND

A variety of blood glucose level measuring apparatuses for measuring a personal blood glucose level (a glucose concentration value in blood) are currently put on sale in the market. Such known blood glucose level measuring apparatus include, e.g., a Self-Monitoring of Blood Glucose (SMBG) apparatus for self-monitoring (measuring) of the blood glucose and a Continuous Glucose Monitoring (CGM) apparatus for continuous glucose monitoring (measuring). The SMBG apparatus measures the blood glucose level in a way that introduces blood extracted from a finger tip by using a needling tool onto a test piece fitted to measurement equipment. The CGM apparatus continuously measures a glucose concentration in interstitial fluid by subcutaneously indwelling a micro sensor including electrodes and enzyme reacting to the glucose. In the CGM apparatus, a sensor is indwelled subcutaneously over a period as long as several days through several weeks, and a blood glucose level is consecutively measured at an interval of several tens of seconds through several minutes. A review about where the CGM apparatus is set on a body of a patient is underway in consideration for relieving a pain and other equivalent symptoms of the patient. It is known to dispose the sensor on an abdominal region, a brachial region, a femoral region and other equivalent regions of a measurement examinee (e.g., Patent document 1). Use of the CGM apparatus enables a blood glucose level to be obtained in real time. The blood glucose level is obtained in real time, and, when in a hypoglycemic state or a hyperglycemic state, it is feasible to give an alarm.

[Patent document 1] Japanese Patent No. 5904500

### SUMMARY

A diabetic patient might, when entering a hypoglycemic state, suddenly go into a comatose state, resulting in a risk of death in a worst case. When a motion quantity (kinetic momentum) is large, (i.e. when a patient's activity level is high) a blood glucose level decreases faster than when the motion quantity (activity level) is small, and the diabetic patient has a possibility of entering the hypoglycemic state without being aware of this state. The diabetic patient himself or herself is unable to self-measure a blood glucose status when sleeping and may enter a nighttime hypoglycemic state (the hypoglycemic state when sleeping) without being aware of this state, and there exists a risk of becoming comatose. It is an aim of at least preferred embodiments of the invention, which is devised in view of such circumstances, to predict fluctuations of a concentration value of a specified substance, which take account of a user's activity status.

According to an aspect of the invention, a measuring apparatus includes: a measurement unit configured to measure a concentration value of a specified substance contained in a sample; an acquiring unit configured to acquire information about a user's activity status; a calculation unit configured to calculate a concentration value of the specified substance within a second period ranging from a present time to a second predetermined time after the present time, based on a concentration value of the specified substance within a first period ranging from a first predetermined time before the present time to the present time and the information about the user's activity status within the first period; and an informing unit configured to inform a user of the information about the concentration value of the specified substance when the concentration value of the specified substance within the second period does not fall within a range of a predetermined concentration value.

The aspect described above may be attained by causing a program to be run by a computer. To be specific, the aspect described above may be provided as a program to be run by the computer, or as a computer readable recording medium on which the program is recorded. The aspect described above may also be provided as a method executed by the computer. The aspect described above may further be provided as a system including the measuring apparatus.

According to the at least preferred embodiments of the invention, it is feasible to predict the fluctuations of the concentration value of the specified substance, which take account of the user's activity status.

### BRIEF DESCRIPTION OF DRAWINGS

Certain preferred embodiments of the present invention will now be described in more detail by way of example only and with reference to the accompanying drawings in which:
FIG. 1A is a view of a configuration of a measuring system according to a first embodiment;
FIG. 1B is a view of a configuration of the measuring system according to a first embodiment;
FIG. 2 is a block diagram of a configuration of a transmitting apparatus according to the first embodiment;
FIG. 3 is a block diagram of a configuration of a receiving apparatus according to the first embodiment;
FIG. 4 is a graphic chart illustrating one example of a glucose concentration value in an interstitial fluid, measurement time, activity factor data and detection time;
FIG. 5 is a graphic chart illustrating one example of the glucose concentration value in the interstitial fluid within a first period, and the glucose concentration values in the interstitial fluid within a second period;
FIG. 6 is a flowchart illustrating one example of a measurement process according to the first embodiment;
FIG. 7 is a flowchart illustrating one example of the measurement process according to the first embodiment;
FIG. 8 is a view of a configuration of a measuring system according to a second embodiment;
FIG. 9 is a block diagram of a configuration of a dosage apparatus according to the second embodiment;
FIG. 10 is a flowchart illustrating an example of a measurement process according to the second embodiment; and
FIG. 11 is a flowchart illustrating an example of the measurement process according to the second embodiment.

### DESCRIPTION OF EMBODIMENT

Embodiments will hereinafter be described with reference to the drawings. The following respective embodiments are exemplary, and the present invention is not limited to configurations of the embodiments given below.

### <First Embodiment

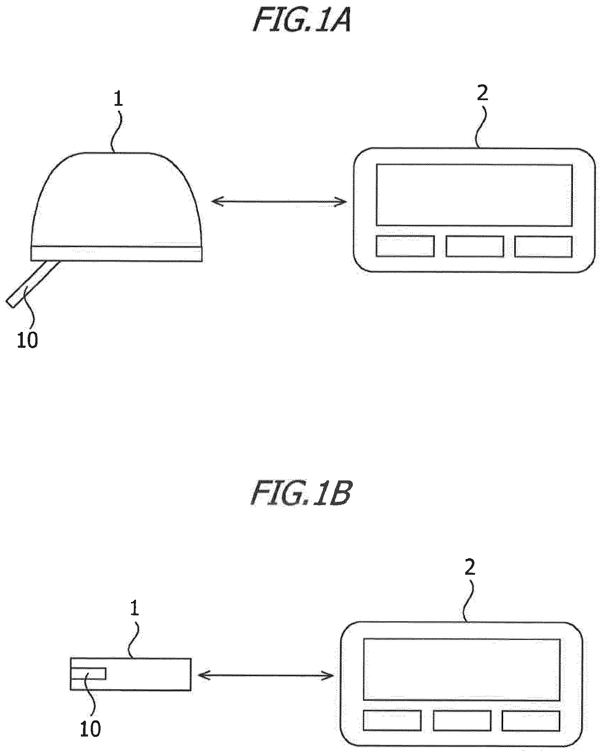

FIG. 1A is a diagram of a configuration of a measuring system according to a first embodiment. The measuring system illustrated in FIG. 1A includes a transmitting apparatus 1 and a receiving apparatus 2. The transmitting apparatus 1 consecutively measures a concentration of a specified substance (measurement target substance) in a sample in vivo, and transmits a measurement result to the receiving apparatus 2. The transmitting apparatus 1 may be used by being attached to regions exemplified by a brachial region, an abdominal region, a gluteal region and a leg region of a user (patient). The transmitting apparatus 1 may also be a Continuous Glucose Monitoring (CGM) apparatus for performing continuous glucose monitoring (measurement). A body fluid exemplified by an interstitial fluid is given as the sample in vivo. The specified substance is exemplified by glucose contained in the interstitial fluid. The specified substance may also be a substance other than glucose.

The receiving apparatus 2 receives a measurement result from the transmitting apparatus 1. The receiving apparatus 2 performs wireless data communications with the transmitting apparatus 1. The receiving apparatus 2 may also perform wired data communications with the transmitting apparatus 1. The transmitting apparatus 1 and the receiving apparatus 2 are configured as separate equipments in the first embodiment, but may instead be provided integrally.

### transmitting Apparatus>

The transmitting apparatus 1 includes a measurement sensor 10 that is used by being implanted into a subcutaneous region of the user. The transmitting apparatus 1 is pasted to a skin of the user by an adhesive tape and other equivalent materials, or is attached to a belt and other equivalent articles, thereby being fitted to the user. The measurement sensor 10 is an electrochemical sensor that measures a specified component (e.g., a concentration of the specified substance) in the sample by utilizing an electrochemical reaction. The measurement sensor 10 is indwelled subcutaneously over a consecutive measurement period as long as, e.g., several days through several weeks, and the transmitting apparatus 1 consecutively measures the glucose concentration in the interstitial fluid. The transmitting apparatus 1 may also be wholly embedded subcutaneously. For example, the embedding type transmitting apparatus 1 illustrated in FIG. 1B is subcutaneously embedded and thus continuously measures a glucose concentration in interstitial fluid. A meaning of "continuous" is that the measurement sensor 10 is subcutaneously indwelled or the transmitting apparatus 1 is subcutaneously embedded, in which state the transmitting apparatus 1 continuously measures the glucose concentration, and encompasses such a mode that the transmitting apparatus 1 measures the glucose concentration at an interval of predetermined time. A measurement frequency of the glucose concentration may be arbitrarily set with respect to the transmitting apparatus 1. For example, the measurement frequency of the glucose concentration may also be set with respect to the transmitting apparatus 1 so that the transmitting apparatus 1 measures the glucose concentration at a frequency of once every several tens of seconds through several minutes.

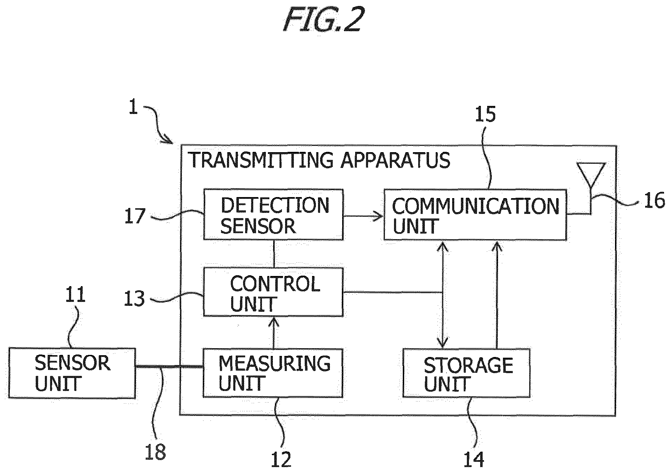

FIG. 2 is a block diagram of a configuration of the transmitting apparatus 1 according to the first embodiment. The transmitting apparatus 1 is a transmitter that transmits various items of data to the receiving apparatus 2. The transmitting apparatus 1 includes a sensor unit 11, a measurement unit 12, a control unit (arithmetic unit) 13, a storage unit 14, a communication unit 15, an antenna 16, and a detection sensor 17. The sensor unit 11 has glucose oxidoreductase exemplified by glucose oxidase (GOD) and glucose dehydrogenase (GDH), and a plurality of electrodes, i.e., a working electrode, a counter electrode, a reference electrode and other equivalent electrodes. The sensor unit 11 is provided on the side of a tip of the measurement sensor 10 illustrated in FIGS. 1A and 1B. The sensor unit 11 is electrically connected to the measurement unit 12 via a wire 18.

The measurement unit 12 is a circuit to measure a signal value (e.g., a response current value) by applying a voltage to the sensor unit 11. When the voltage is applied to between the electrodes (between the working electrode and the counter electrode, or between the working electrode and the reference electrode) of the sensor unit 11, the sensor unit 11 outputs a response current value corresponding to the glucose concentration in the body fluid. The measurement unit 12 measures the response current value outputted from the sensor unit 11 in a way that controls the voltage to be applied to between the electrodes of the sensor unit 11. When the voltage is applied to between the electrodes of the sensor unit 11, the glucose in the body fluid is oxidized by the oxidoreductase, and electrons being thereby extracted are supplied to the working electrode. The measurement unit 12 measures, as the response current value, a quantity of electric charges of the electrons supplied to the working electrode. The measurement unit 12 may convert the response current value into a response voltage value, and may measure, as the response voltage value, the quantity of electric charges of the electrons supplied to the working electrode. The following discussion will deal with a case that the measurement unit 12 measures the response current value. The response current value measured by the measurement unit 12 is sent to the control unit 13.

The control unit 13 controls the measurement unit 12, the storage unit 14, the communication unit 15, and the detection sensor 17. The control unit 13, the storage unit 14 and the communication unit 15 may be provided by: computers each including a Central Processing Unit (CPU), a Random Access Memory (RAM), a Read Only Memory (ROM) and other equivalent hardware components that are provided in the transmitting apparatus 1; respective apparatuses; and programs and other equivalent software components running on the computer. The CPU is also called a processor. It does not mean that the CPU is limited to the single processor, and the CPU may, however, take a multi-processor configuration.

The control unit 13 stores a response current value and time (which will hereinafter be termed "measurement time") when measuring the response current value in the storage unit 14, and transmits the response current value and the measurement time to the communication unit 15. The communication unit 15 sends the response current value and the measurement time to the receiving apparatus 2 via the antenna 16. The communication unit 15 may also send, to the receiving apparatus 2, the response current value and the measurement time that are transmitted from the control unit 13, or the response current value and the measurement time that are stored in the storage unit 14.

The detection sensor 17 detects an activity factor of the user attached with the transmitting apparatus 1, and outputs a signal corresponding to the activity factor of the user. The detection sensor 17 comprises at least one of a motion sensor, an attitude sensor and a vital-sign sensor. The motion sensor is an activity sensor to detect a quantity of motion of the user attached with the transmitting apparatus 1, and, e.g., an acceleration sensor is provided as the motion sensor. The attitude sensor is an activity sensor to detect an attitude of the user attached with the transmitting apparatus 1, and, e.g., a gyro sensor is given as the attitude sensor. The vital-sign sensor is an activity sensor to measure vital-sign data of the user attached with the transmitting apparatus 1, and, e.g., one or more of of a temperature sensor, a pulse sensor, a heart rate sensor (heartbeat sensor), a pulse wave sensor and a blood pressure sensor is provided as the vital-sign sensor. The temperature sensor measures a body temperature of the user attached with the transmitting apparatus 1. The pulse sensor measures a pulse rate of the user attached with the transmitting apparatus 1. The heart rate sensor measures a heart rate (heartbeat rate) of the user attached with the transmitting apparatus 1. The pulse wave sensor measures pulse waves of the user attached with the transmitting apparatus 1. The blood pressure sensor measures a blood pressure level of the user attached with the transmitting apparatus 1.

The user's activity factor detected by the detection sensor 17 is sent as activity factor data to the control unit 13. The control unit 13 stores, in the storage unit 14, the activity factor data and time (which will hereinafter be referred to as "detection time") when the activity factor is detected, and transmits the activity factor data and the detection time to the communication unit 15. The communication unit 15 transmits the activity factor data and the detection time to the receiving apparatus 2 via the antenna 16. The activity factor data includes at least one of motion quantity data (acceleration data), attitude data (angular velocity data and angular acceleration data), body temperature data, pulse rate data, heart rate (heartbeat rate) data, pulse wave data and blood pressure level data. The activity factor data is one example of a "signal".

### <Receiving Apparatus>

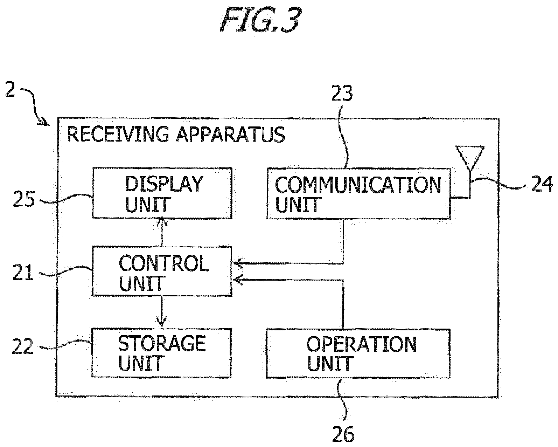

FIG. 3 is a block diagram illustrating a configuration of the receiving apparatus 2 according to the first embodiment. The receiving apparatus 2 receives the various items of data from the transmitting apparatus 1, and displays the received data. The receiving apparatus 2 includes a control unit (arithmetic unit) 21, a storage unit 22, a communication unit 23, an antenna 24, a display unit 25, and an operation unit 26. The control unit 21 controls the storage unit 22, the communication unit 23, and the display unit 25. The control unit 21, the storage unit 22, and the communication unit 23 may be provided by: computers each including the CPU, the RAM, the ROM and other equivalent hardware components that are provided in the receiving apparatus 2; respective apparatuses; and programs and other equivalent software components running on the computer.

The display unit 25 has a display and displays various types of information and messages on this display. The display unit 25 displays a measurement result and an error on the display, and also displays operation procedures, operation statuses and other equivalent items when setting is done. The display of the display unit 25 is exemplified by a liquid crystal display apparatus, a plasma display panel, a Cathode Ray Tube (CRT) display, or an Electroluminescence (EL) panel. The display unit 25 may have a voice output unit to output voices and sounds. The operation unit 26 includes a variety of operation buttons, a touch panel and other equivalent components, and accepts the various types of information from the user.

The communication unit 23 receives the response current value, the measurement time, the activity factor data and the detection time from the transmitting apparatus 1 via the antenna 24, and sends the response current value, the measurement time, the activity factor data and the detection time to the control unit 21. The control unit 21 converts the response current value into the glucose concentration value by referring to calibration curve data stored in the storage unit 22. The calibration curve data indicating a correspondence relation between the response current value and the glucose concentration in the interstitial fluid is pre-stored in the storage unit 22. The calibration curve data is pre-stored as, e.g., a mathematical expression and/or a correspondence table in the storage unit 22. The control unit 21 calculates the glucose concentration value in the interstitial fluid, based on the response current value and the calibration curve data. The control unit 21 is one example of a "measurement unit".

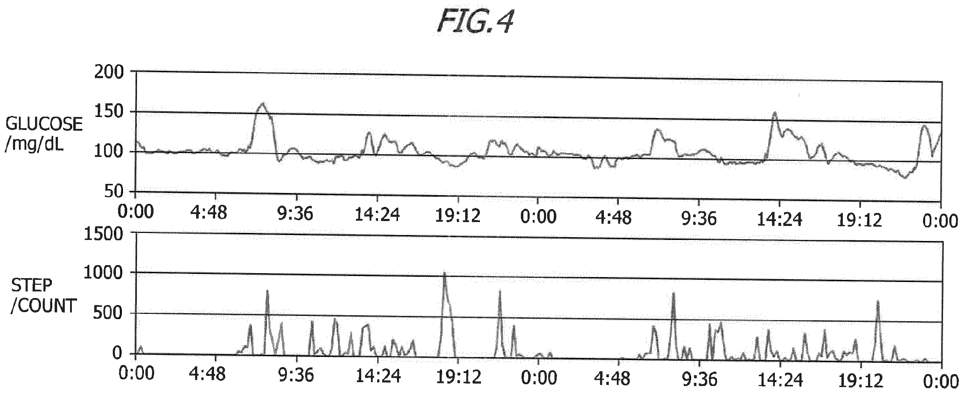

The control unit 21 stores, in the storage unit 22, the glucose concentration value in the interstitial fluid, the measurement time, the activity factor data and the detection time. FIG. 4 is a graphic chart illustrating one example of the glucose concentration value in the interstitial fluid, the measurement time, the activity factor data and the detection time. An upper graph of FIG. 4 indicates variations in glucose concentration value in the interstitial fluid together with the measurement time. A lower graph of FIG. 4 indicates the user's activity quantity together with the detection time. The user's activity quantity indicated by the lower graph of FIG. 4 is a number of steps per unit time. The number of steps of the user is calculable from the acceleration data detected by the acceleration sensor.

The control unit 21 acquires information about activity statuses of the user, based on the activity factor data. The user's activity statuses include statuses of body motions (a variety of events) of the user, and/or body statuses (body temperature and other equivalent statuses) pertaining to the user's life support (vital-signs). The information about the user's activity statuses includes the user's activity quantity and/or the user's vital-sign data (biometric information). The control unit 21 is one example of an "acquiring unit". The control unit 21 may calculate the user's activity quantity from the motion quantity data. For example, the control unit 21 may also calculate the user's activity quantity per unit time from the motion quantity data. The user's activity quantity tends to be small when the user is in a sleeping status but large when the user is not in the sleeping status (non-sleeping status). The control unit 21 determines that the user's activity status is the first status when the user's activity quantity falls within a range of a predetermined quantity. The sleeping status of the user is given as one example of the first status. The control unit 21, when the user's activity quantity does not fall within the predetermined quantity range, determines that the user's activity status is the second status. The non-sleeping status of the user is given as one example of the second status. The control unit 21 may determine that the user's activity status is the first status when the user's activity quantity does not fall within the predetermined quantity range. The control unit 21 may also determine that the user's activity status is the second status when the user's activity quantity falls within the predetermined quantity range.

The control unit 21 calculates information representing a user's attitude from the attitude data. For example, the control unit 21 may also calculate the information representing the user's attitude per unit time from the attitude data. The control unit 21 determines that the user's activity status is the first status when the user's attitude is a predetermined status during a fixed period. A lying status of the user is given as one example of the predetermined status. The control unit 21 may numerize the user's attitude and may calculate the numerized value as the user's activity quantity. For instance, the control unit 21 may reckon a standing status of the user as "1" and the lying status of the user as "0".

The control unit 21 calculates a body temperature (temperature value) from the body temperature data. The body temperature of the user is one example of "vital-sign information of the user". The control unit 21 determines that the user's activity status is the first status when the body temperature of the user falls within a range of a predetermined temperature. The user's body temperature tends to rise when the user is exercising and eating, but tends to reduce when the user is sleeping. The control unit 21 may determine, based on an average body temperature of the user, whether the user's body temperature falls within a predetermined temperature range.

The control unit 21 calculates a user's pulse rate from pulse rate data. The user's pulse rate is one example of the "vital-sign information of the user". The control unit 21 determines that the activity status of the user is the first status when the user's pulse rate falls within a range of a predetermined pulse rate. The user's pulse rate tends to rise when the user is exercising and eating, but tends to reduce when the user is sleeping. The control unit 21 may determine, based on an average pulse rate of the user, whether the user's pulse rate falls within the predetermined pulse rate range.

The control unit 21 calculates the user's heart rate from heart rate data. The user's heart rate is one example of the "vital-sign information of the user". The control unit 21 determines that the user's activity status is the first status when the user's heart rate falls within a range of a predetermined heart rate. The user's heart rate tends to rise when the user is exercising and eating, but tends to reduce when the user is sleeping. The control unit 21 may determine, based on an average heart rate of the user, whether the user's heart rate falls within a predetermined heart rate range.

The control unit 21 calculates a waveform of pulse waves of the user from pulse wave data. The waveform of the pulse waves of the user is one example of the "vital-sign information of the user". The control unit 21 may determine whether the user's activity status is the first status by comparing the waveform of the pulse waves of the user with a waveform of predetermined pulse waves being pre-stored in the storage unit 22. The storage unit 22 may also store the waveform of the predetermined pulse waves. The control unit 21 may calculate the user's pulse rate by making a frequency analysis of the waveform of the pulse waves of the user.

The control unit 21 calculates a blood pressure level of the user from blood pressure level data. The blood pressure level of the user is one example of the "vital-sign information of the user". The control unit 21 determines that the user's activity status is the first status when the blood pressure level of the user falls within a range of a predetermined value. The blood pressure level of the user tends to rise when the user is exercising and eating, but tends to reduce when the user is sleeping. The control unit 21 may determine, based on an average blood pressure level of the user, whether the blood pressure level of the user falls within the predetermined value range.

The control unit 21 calculates a predicted glucose concentration value in the interstitial fluid within a second period ranging from the present time to second predetermined time on the basis of a glucose concentration value in the interstitial fluid within a first period ranging from first predetermined time to the present time and information about the user's activity status within the first period. The first predetermined time is time (past time) before the present time. The second predetermined time is time (future time) after the present time. A length of the first period may be equal to a length of the second period. The length of the first period may be longer than the length of the second period. The length of the second period may be longer than the length of the first period. The glucose concentration value in the interstitial fluid within the first period may be a glucose concentration value in the interstitial fluid at an arbitrary point within the first period. The glucose concentration value in the interstitial fluid within the first period may include glucose concentration values in the interstitial fluid at a plurality of arbitrary points within the first period. The glucose concentration value in the interstitial fluid within the first period may be an average value of the glucose concentration values in the interstitial fluid at the plurality of arbitrary points within the first period. The glucose concentration value in the interstitial fluid within the first period may include glucose concentration values in the interstitial fluid for arbitrary consecutive time spans within the first period. The glucose concentration value in the interstitial fluid within the first period may be an average value of the glucose concentration values in the interstitial fluid for arbitrary consecutive time spans within the first period. The information about the user's activity status within the first period may be information about the user's activity status at an arbitrary point within the first period. The information about the user's activity status within the first period may be information about the user's activity statuses at a plurality of arbitrary points within the first period. The information about the user's activity status within the first period may be an average value of the user's activity statuses at a plurality of arbitrary points within the first period. The information about the user's activity status within the first period may be information about the user's activity statuses for arbitrary consecutive time spans within the first period. The information about the user's activity status within the first period may be an average value of the user's activity statuses for arbitrary consecutive time spans within the first period.

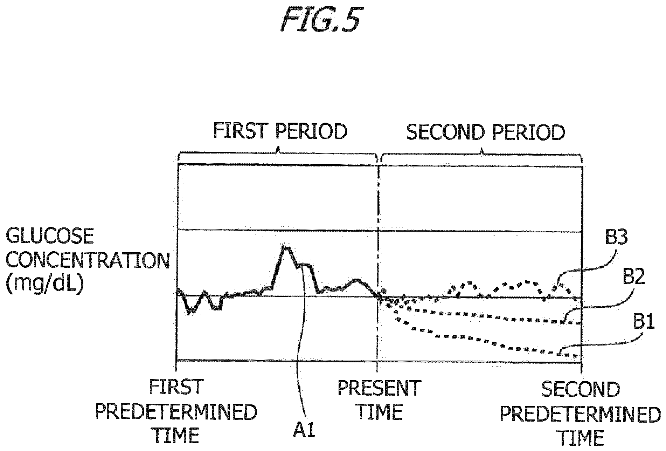

FIG. 5 is a graphic chart illustrating one example of the glucose concentration value in the interstitial fluid within the first period, and the glucose concentration values in the interstitial fluid within the second period. In FIG. 5, an axis of abscissa indicates the time, and an axis of ordinate indicates the glucose concentration value in the interstitial fluid. In FIG. 5, a solid line A1 represents the glucose concentration value in the interstitial fluid within the first period, and dotted lines B1 - B3 represent the glucose concentration values in the interstitial fluid within the second period. The glucose concentration value in the interstitial fluid is affected by the user's activity status and the user's activity quantity. For example, the glucose concentration value in the interstitial fluid after the present time fluctuates corresponding to the user's activity status and the user's activity quantity before the present time.

The dotted line B1 in FIG. 5 represents the glucose concentration value in the interstitial fluid when the user's status within the first period is the non-sleeping status and when the user's activity quantity within the first period is equal to or larger than a threshold value. As indicated by the dotted line B1 in FIG. 5, the predicted glucose concentration value in the interstitial fluid within the second period sharply decreases due to the user's high activity quantity before the present time. The dotted line B2 in FIG. 5 represents the glucose concentration value in the interstitial fluid when the user's status within the first period is the sleeping status. As indicated by the dotted line B2 in FIG. 5, the predicted glucose concentration value in the interstitial fluid within the second period gently decreases because of the user's status before the present time being the sleeping status. The dotted line B3 in FIG. 5 represents the glucose concentration value in the interstitial fluid when the user's status within the first period is the non-sleeping status and when the user's activity quantity within the first period is less than the threshold value. As indicated by the dotted line B3 in FIG. 5, the predicted glucose concentration value in the interstitial fluid within the second period fluctuates minutely because of the user's activity quantity before the present time being small.

The storage unit 22 stores a plurality of prediction algorithms and information about the user's activity statuses in a state of being associated with each other. The prediction algorithm is one example of a "calculation algorithm". The plurality of prediction algorithms and the information about the user's activity statuses may also be stored in another storage unit different from the storage unit 22. The storage unit 22 is one example of a "first storage unit". The control unit 21 selects one of the plurality of prediction algorithms stored in the storage unit 22, based on the information about the user's activity status within the first period. The prediction algorithm is an algorithm for predicting the glucose concentration value in the interstitial fluid within the second period in a way that takes account of the information about the user's activity status within the first period. The control unit 21 predicts the glucose concentration value in the interstitial fluid within the second period from the glucose concentration value in the interstitial fluid within the first period by using the selected prediction algorithm. The control unit 21 is one example of a "calculation unit". The predicted glucose concentration value in the interstitial fluid within the second period may be a glucose concentration value in the interstitial fluid at an arbitrary point within the second period. The predicted glucose concentration value in the interstitial fluid within the second period may include glucose concentration values in the interstitial fluid at a plurality of arbitrary points within the second period. The predicted glucose concentration value in the interstitial fluid within the second period may be an average value of the glucose concentration values in the interstitial fluid at the plurality of arbitrary points within the second period. The predicted glucose concentration value in the interstitial fluid within the second period may include glucose concentration values in the interstitial fluid for arbitrary consecutive time spans within the second period. The predicted glucose concentration value in the interstitial fluid within the second period may be an average value of the glucose concentration values in the interstitial fluid for arbitrary consecutive time spans within the second period.

The control unit 21 determines whether the predicted glucose concentration value in the interstitial fluid within the second period falls within a range of a predetermined concentration value. When the predicted glucose concentration value in the interstitial fluid within the second period does not fall within the predetermined concentration value range, the control unit 21 informs the user of the information about the glucose concentration value in the interstitial fluid. When a minimum predicted value or an average predicted value of the glucose concentration values in the interstitial fluid within the second period is equal to or smaller than a first reference value, the control unit 21 informs the user of an alarm message. When a maximum predicted value or the average predicted value of the glucose concentration values in the interstitial fluid within the second period is equal to or larger than a second reference value, the control unit 21 informs the user of the alarm message. The control unit 21 is one example of an "informing unit". For example, the control unit 21 may output the alarm message to the display unit 25 and the display unit 25 may display the alarm message, or the display unit 25 may output a voice of the alarm message. The display unit 25 may also output the voice of the alarm message along with displaying the alarm message.

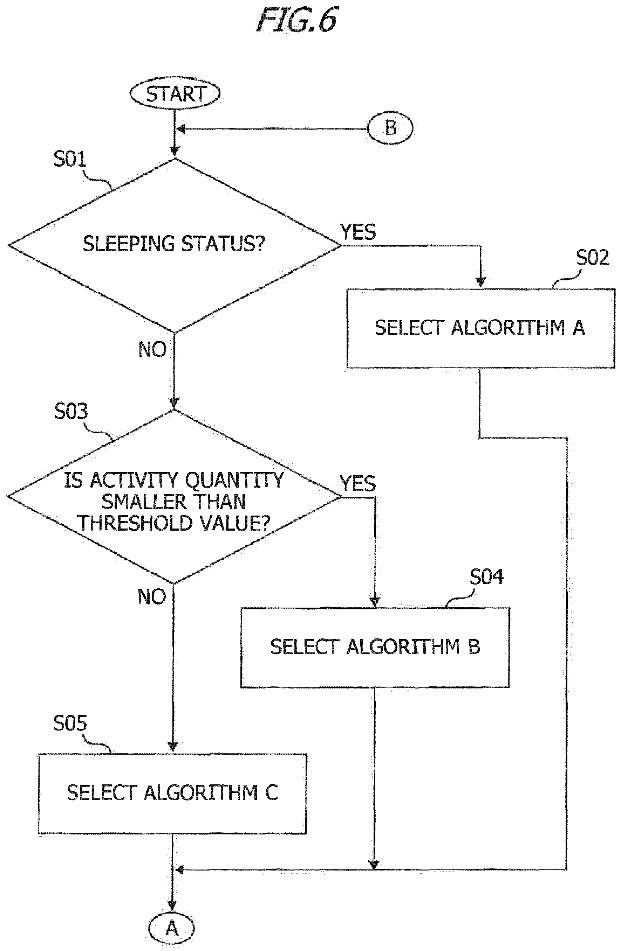

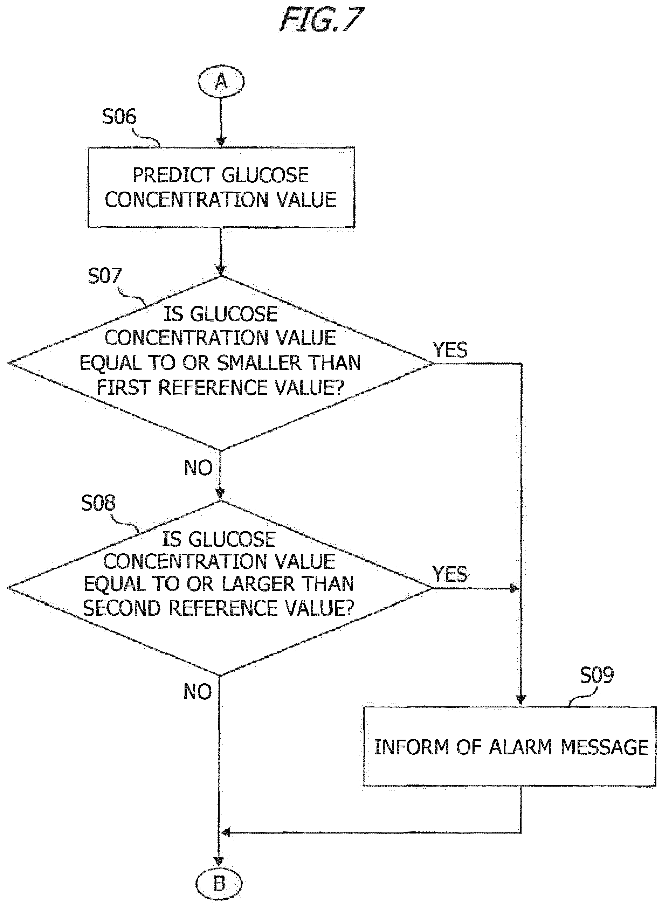

FIGS. 6 and 7 are flowcharts illustrating one example of a measuring process according to the first embodiment. The transmitting apparatus 1 and the receiving apparatus 2 are powered ON, and thereafter the transmitting apparatus 1 is fitted to a body surface of the user and is thus attached to the user. The transmitting apparatus 1 and the receiving apparatus 2 are initialized, thereby starting a flow (processing flow) illustrated in FIGS. 6 and 7. The transmitting apparatus 1 may be powered ON after the transmitting apparatus 1 has been fitted to the body surface of the user. Upon the start of the flow illustrated in FIGS. 6 and 7, the transmitting apparatus 1 transmits a response current value, measurement time, activity factor data and detection time to the receiving apparatus 2, while the receiving apparatus 2 receives the response current value, the measurement time, the activity factor data and the detection time.

In step S01, the control unit 21 determines, based on the activity factor data, whether the user's activity status within the first period is the sleeping status. The processing diverts to step S02 when the user's activity status within the first period is the sleeping status. Whereas when the user's activity status within the first period is not the sleeping status, i.e., when the user's activity status within the first period is the non-sleeping status, the processing proceeds to step S03.

In step S02, the control unit 21 selects a prediction algorithm A stored in the storage unit 22. The prediction algorithm A is an algorithm used when the user's status within the first period is the sleeping status. After executing the process in step S02, the processing proceeds to step S06. In step S06, the control unit 21 calculates the predicted glucose concentration value in the interstitial fluid within the second period from the glucose concentration value in the interstitial fluid within the first period by using the selected prediction algorithm A.

In step S03, the control unit 21 determines, based on the activity factor data, whether the user's activity quantity within the first period is smaller than the threshold value. When the user's activity quantity within the first period is smaller than the threshold value, the processing diverts to step S04. Whereas when the user's activity quantity within the first period is not smaller than the threshold value, i.e., when the user's activity quantity within the first period is equal to or larger than the threshold value, the processing proceeds to step S05.

In step S04, the control unit 21 selects a prediction algorithm B stored in the storage unit 22. The prediction algorithm B is an algorithm used when the user's status within the first period is the non-sleeping status and when the user's activity quantity within the first period is smaller than the threshold value. After executing the process in step S04, the processing proceeds to step S06. In step S06, the control unit 21 calculates the predicted glucose concentration value in the interstitial fluid within the second period from the glucose concentration value in the interstitial fluid within the first period by employing the selected prediction algorithm B.

In step S05, the control unit 21 selects a prediction algorithm C stored in the storage unit 22. The prediction algorithm C is an algorithm used when the user's status within the first period is the non-sleeping status and when the user's activity quantity within the first period is equal to or larger than the threshold value. After executing the process in step S05, the processing proceeds to step S06. In step S06, the control unit 21 calculates the predicted glucose concentration value in the interstitial fluid within the second period from the glucose concentration value in the interstitial fluid within the first period by using the selected prediction algorithm C.

In step S07, the control unit 21 determines whether the minimum predicted value of the glucose concentration values in the interstitial fluid within the second period is equal to or smaller than the first reference value. When the minimum predicted value of the glucose concentration values in the interstitial fluid within the second period is not equal to or smaller than the first reference value, the processing proceeds to step S08. Whereas when the minimum predicted value of the glucose concentration values in the interstitial fluid within the second period is equal to or smaller than the first reference value, the processing diverts to step S09. In step S09, the control unit 21 informs the user of the alarm message. The alarm message may be a message indicating that the minimum value of the glucose concentration values in the interstitial fluid is equal to or smaller than the first reference value, and may also be a message indicating that the user is in (or may go into) a hypoglycemic state. The control unit 21 may inform the user of alarms as a substitute for the alarm message. The alarms include a visual alarm, an audio alarm, a vibrational alarm or the like. In step S07, the control unit 21 may determine whether an average predicted value of the glucose concentration values in the interstitial fluid within the second period is equal to or smaller than the first reference value. When the average predicted value of the glucose concentration values in the interstitial fluid within the second period is not equal to or smaller than the first reference value, the processing proceeds to step S08. Whereas when the average predicted value of the glucose concentration values in the interstitial fluid within the second period is equal to or smaller than the first reference value, the processing diverts to step S09.

In step S08, the control unit 21 determines whether a maximum predicted value of the glucose concentration values in the interstitial fluid within the second period is equal to or larger than the second reference value. When the maximum predicted value of the glucose concentration values in the interstitial fluid within the second period is not equal to or larger than the second reference value, the processing loops back to step S01. For example, after an elapse of predetermined time, the control unit 21 executes the process in S01. Whereas when the maximum predicted value of the glucose concentration values in the interstitial fluid within the second period is equal to or larger than the second reference value, the processing diverts to step S09. In step S09, the control unit 21 informs the user of the alarm message. The alarm message may be a message indicating that the maximum value of the glucose concentration values in the interstitial fluid is equal to or larger than the second reference value, and may also be a message indicating that the user is in a hyperglycemic state. The control unit 21 may also inform the user of the alarms as the substitute for the alarm message. In step S08, the control unit 21 may determine whether an average predicted value of the glucose concentration values in the interstitial fluid within the second period is equal to or larger than the second reference value. When the average predicted value of the glucose concentration values in the interstitial fluid within the second period is not equal to or larger than the second reference value, the processing loops back to step S01. Whereas when the average predicted value of the glucose concentration values in the interstitial fluid within the second period is equal to or larger than the second reference value, the processing proceeds to step S09.

According to the first embodiment, there is calculated the predicted glucose concentration value in the interstitial fluid within the second period ranging from the present time to the second time after the present time, corresponding to the user's activity status within the first period ranging from the first predetermined time before the present time to the present time. The user is informed of the information about the glucose concentration value in the interstitial fluid when the predicted glucose concentration value in the interstitial fluid within the second period does not fall within the range of the predetermined concentration value. The user's activity status includes at least one of a body motion status of the user and a body status pertaining to a life support of the user. The first embodiment therefore enables a prediction of the fluctuations of the glucose concentration value, which takes account of the body motion status of the user and the body status pertaining to the life support (vital-signs) of the user.

When the minimum predicted value or the average predicted value of the glucose concentration values in the interstitial fluid within the second period is equal to or smaller than the first reference value, the user is informed of the alarm message and is thereby enabled to recognize that the glucose concentration value in the interstitial fluid within the second period may decrease. A treatment for the user to avoid entering the hypoglycemic state is conducted, thereby reducing a risk that the user enters the hypoglycemic state. The user is therefore enabled to avoid entering the hypoglycemic state without being aware of this state and to avoid entering a nighttime hypoglycemic state without being aware of this state. When the maximum predicted value or the average predicted value of the glucose concentration values in the interstitial fluid within the second period is equal to or larger than the second reference value, the user is informed of the alarm message and is thereby enabled to recognize that the glucose concentration value in the interstitial fluid within the second period may increase. A treatment for the user to avoid entering the hyperglycemic state is conducted, thereby reducing a risk that the user enters the hyperglycemic state. The user is therefore enabled to avoid entering the hyperglycemic state without being aware of this state and to avoid entering a nighttime hyperglycemic state (hyperglycemia state when sleeping) without being aware of this state.

According to the first embodiment, one of the plural prediction algorithms stored in the storage unit 22 is selected based on the information about the user's activity status within the first period. The predicted glucose concentration value in the interstitial fluid within the second period is calculated from the glucose concentration value in the interstitial fluid within the first period by using the selected prediction algorithm. The proper prediction algorithm is selected by obtaining the information about the user's activity status within the first period, thereby enabling the prediction of the glucose concentration value in the interstitial fluid within the second period.

The measurement system including the transmitting apparatus 1 and the receiving apparatus 2 has been so far described, and the first embodiment is not, however, limited to this example. The transmitting apparatus 1 and the receiving apparatus 2 may be configured integrally as a measurement apparatus. Any one of the transmitting apparatus 1 and the receiving apparatus 2 may also be configured as the measurement apparatus. The control unit 13 of the transmitting apparatus 1 may function as at least one of the "measurement unit", the "acquiring unit", the "calculation unit", and the "informing unit". The control unit 21 of the receiving apparatus 2 may function as at least one of the "measurement unit", the "acquiring unit", the "calculation unit", and the "informing unit". The storage unit 14 of the transmitting apparatus 1 may store the plurality of prediction algorithms and the information about the user's activity status. The storage unit 14 is one example of the "first storage unit".

### <Second Embodiment

A second embodiment will be described. The following discussion will be focused on different points between the first embodiment and the second embodiment, and the same components in the second embodiment as those in the first embodiment are marked with the same numerals and symbols as those in the first embodiment, while their repetitive explanations are omitted.

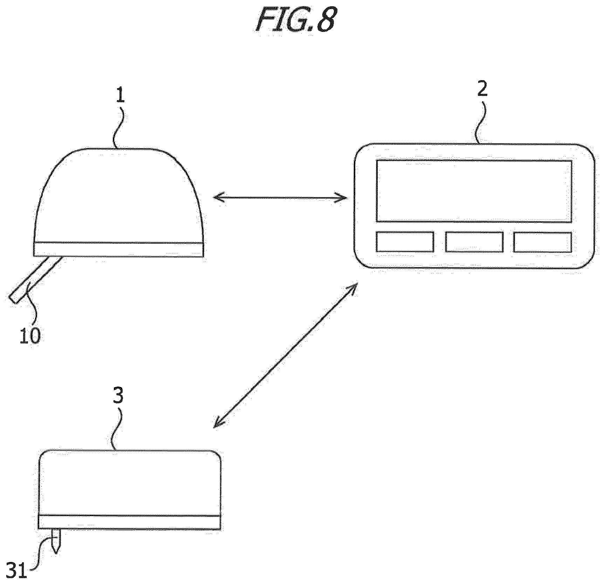

FIG. 8 is a diagram of a configuration of the measuring system according to the second embodiment. The measuring system illustrated in FIG. 8 includes the transmitting apparatus 1, the receiving apparatus 2 and a dosage apparatus 3. The transmitting apparatus 1 and the receiving apparatus 2 according to the second embodiment are the same as those of the first embodiment. The dosage apparatus 3 is a medicine supply apparatus to consecutively (continuously) or intermittently supply a medicine in vivo. The dosage apparatus 3 may be used by being attached to regions exemplified by the abdominal region, the brachial region and the gluteal region. The dosage apparatus 3 may take any one of a patch (paste) type and a tube type. The medicines include insulin and glucagon. The dosage apparatus 3 performs wireless data communications with the receiving apparatus 2. The dosage apparatus 3 may perform wired data communications with the receiving apparatus 2. The transmitting apparatus 1 and the receiving apparatus 2, though configured as separate pieces of equipment in the second embodiment, may also be provided as an integral piece of equipment. The transmitting apparatus 1 and the dosage apparatus 3, though configured as separate pieces of equipment in the second embodiment, may also be provided as an integral piece of equipment. The transmitting apparatus 1, the receiving apparatus 2 and the dosage apparatus 3 may also be provided as an integral piece of equipment.

### <Dosage Apparatus>

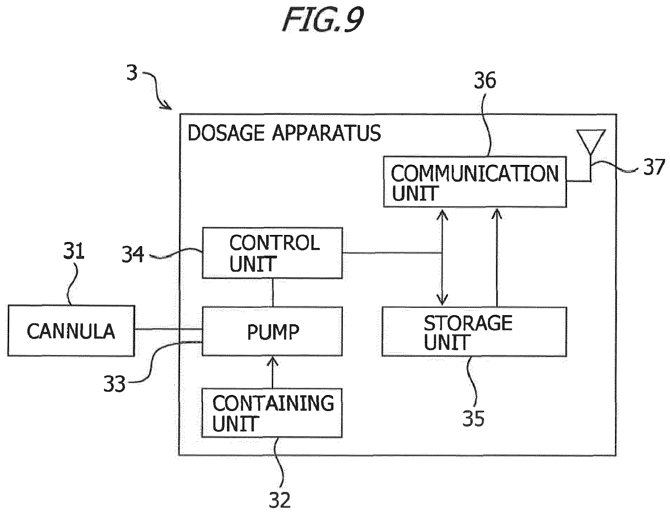

The dosage apparatus 3 includes a cannula (insertion unit) 31 used by being implanted into a subcutaneous region of the user. The dosage apparatus 3 is pasted to a skin of the user by an adhesive tape and other equivalent materials, or is attached to a piece of clothing, a belt and other equivalent articles, thereby being attached to the user. FIG. 9 is a block diagram of a configuration of the dosage apparatus 3 according to the second embodiment. The dosage apparatus 3 includes, the cannula 31, a containing unit 32, a pump 33, a control unit (arithmetic unit) 34, a storage unit 35, a communication unit 36, and an antenna 37. The cannula 31 is connected to the pump 33. The containing unit 32 contains medicines. The containing unit 32 may contain plural types of medicines. A plurality of containing units 32 may also be provided in the dosage apparatus 3. For example, one of the plural containing units 32 may contain insulin, while another of the plural containing units 32 may also contain glucagon.

The pump 33 is actuated by, e.g., a motor and other equivalent devices. The pump 33 is actuated to feed the medicine within the containing unit 32 to the cannula 31, whereby the medicine is dosed in vivo. The pump 33 is one example of a "dosage unit". A plurality of pumps 33 may be provided in the dosage apparatus 3. For instance, one of the plural pumps 33 may be an insulin pump, while another of the plural pumps 33 may also be a glucagon pump. The control unit 34 controls the pump 33, the storage unit 35 and the communication unit 36. The control unit 34 receives various items of data from the receiving apparatus 2 via the communication unit 36 and the antenna 37. The control unit 34 transmits the various items of data to the receiving apparatus 2 via the communication unit 36 and the antenna 37. The control unit 34, the storage unit 35 and the communication unit 36 may be provided by: computers each including the CPU, the RAM, the ROM and other equivalent hardware components that are provided in the dosage apparatus 3; respective apparatuses; and programs and other equivalent software components running on the computer.

The receiving apparatus 2 according to a second embodiment will be described. A plurality of dosage algorithms is stored in the storage unit 22. The plurality of dosage algorithms may also be stored in another storage unit different from the storage unit 22. The storage unit 22 or another storage unit is one example of a "second storage unit". The dosage algorithm is, e.g., a processing procedure for dosing a medicine in vivo. The dosage algorithm includes a type of the medicine to be dosed, a dose quantity of the medicine (unit/min), a dosing period of the medicine and dosing timing of the medicine (dosing time zone). The control unit 21 selects one of the plural dosage algorithms stored in the storage unit 22 on the basis of the predicted glucose concentration value in the interstitial fluid within the second period. The storage unit 22 stores the plurality of dosage algorithms and the glucose concentration values in the interstitial fluid in a state of associating the plurality of dosage algorithms with the glucose concentration values in the interstitial fluid.

The control unit 21 determines whether the predicted glucose concentration value in the interstitial fluid within the second period falls within the range of the predetermined concentration value. When the predicted glucose concentration value in the interstitial fluid within the second period does not fall within the range of the predetermined concentration value, the control unit 21 controls a pump 33 to dose the medicine in vivo. The control unit 21 may control the pump 33 via a control unit 34. When the predicted minimum value or the predicted average value of the glucose concentration values in the interstitial fluid within the second period is equal to or smaller than the first reference value, the control unit 21 controls the pump 33 to dose the medicine in vivo. When the maximum predicted value or the average predicted value of the glucose concentration values in the interstitial fluid within the second period is equal to or larger than the second reference value, the control unit 21 controls the pump 33 to dose the medicine in vivo. The pump 33 is actuated to feed the medicine within a containing unit 32 to a cannula 31, thereby dosing the medicine in vivo.

Upon dosing the medicine in vivo, the glucose concentration value in vivo increases or decreases due to an effect of the medicine. The glucose concentration value in the blood and the glucose concentration value in the interstitial fluid are thereby increased or decreased. Upon dosing the insulin in vivo, the glucose concentration value in the blood and the glucose concentration value in the interstitial fluid decrease. Upon dosing the glucagon in vivo, the glucose concentration value in the blood and the glucose concentration value in the interstitial fluid increase. For instance, when the glucose concentration in the interstitial fluid has a high value, the insulin is dosed in vivo, whereby the glucose concentration value in the blood and the glucose concentration value in the interstitial fluid decreases. For example, when the glucose concentration in the interstitial fluid has a low value, the glucagon is dosed in vivo, whereby the glucose concentration value in the blood and the glucose concentration value in the interstitial fluid increases. The medicine is thus dosed in vivo, and the glucose concentration value in the blood and the glucose concentration in the interstitial fluid is thereby enabled to keep a desired value.

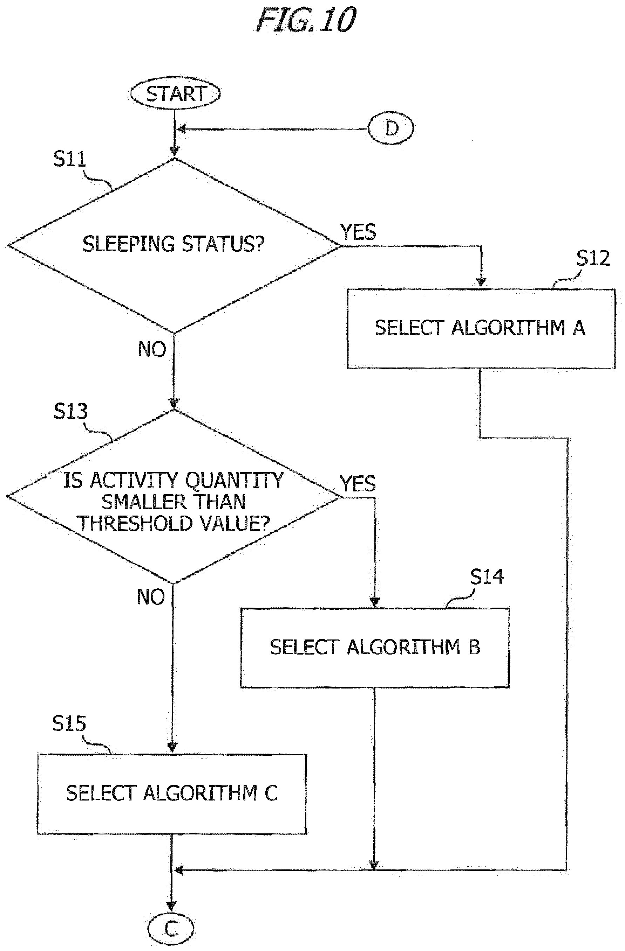

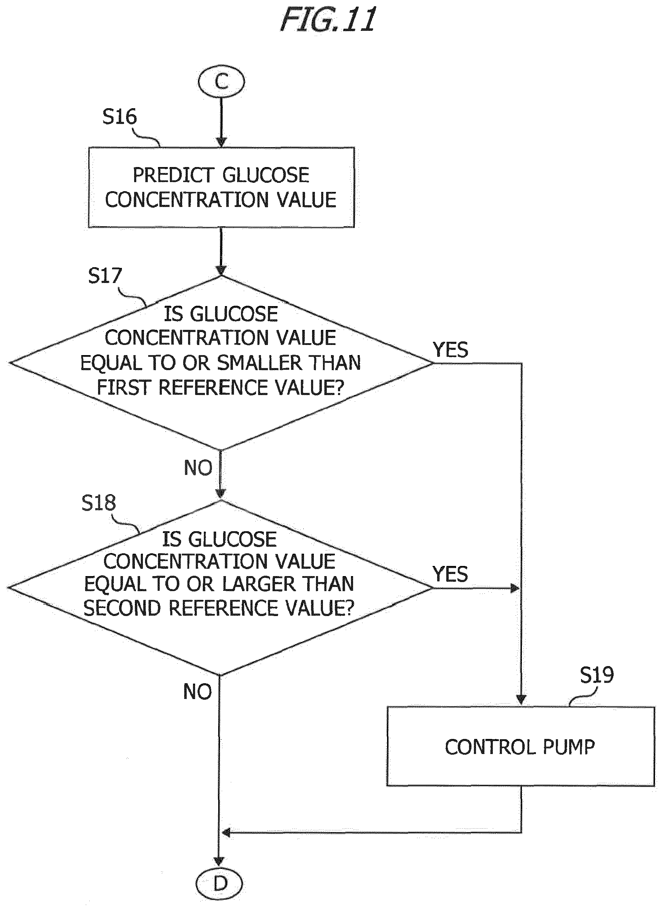

FIGS. 10 and 11 are flowcharts illustrating one example of the measurement process according to the second embodiment. A condition for starting a flow illustrated in FIGS. 10 and 11 is the same as the condition for starting the flow depicted in FIGS. 6 and 7. Upon a start of the flow illustrated in FIGS. 10 and 11, the transmitting apparatus 1 transmits the response current value, the measurement time, the activity factor data and the detection time to the receiving apparatus 2, while the receiving apparatus 2 receives the response current value, the measurement time, the activity factor data and the detection time. Processes in steps S11 - S15 of FIG. 10 are the same as the processes in steps S01 - S05 of FIG. 6. A process in step S16 of FIG. 11 is the same as the process in step S06 of FIG. 7.

Processes in steps S17 - S19 of FIG. 11 will be described. In step S17, the control unit 21 determines whether the minimum predicted value of the glucose concentration values in the interstitial fluid within the second period is equal to or smaller than the first reference value. When the minimum predicted value of the glucose concentration values in the interstitial fluid within the second period is not equal to or smaller than the first reference value, the processing proceeds to step S18. Whereas when the minimum predicted value of the glucose concentration values in the interstitial fluid within the second period is equal to or smaller than the first reference value, the processing diverts to step S19. In step S19, the control unit 21 selects glucagon as the type of medicine, and controls the pump 33 to dose the medicine in vivo. The control unit 21 may select one of the plural dosage algorithms stored in the storage unit 22, based on the predicted glucose concentration value in the interstitial fluid within the second period. The control unit 21 may also control the pump 33 to dose the medicine in vivo by using the selected dosage algorithm. In step S17, the control unit 21 may determine whether the predicted average value of the glucose concentration values in the interstitial fluid within the second period is equal to or smaller than the first reference value. When the predicted average value of the glucose concentration values in the interstitial fluid within the second period is not equal to or smaller than the first reference value, the processing proceeds to step S18. Whereas when the predicted average value of the glucose concentration values in the interstitial fluid within the second period is equal to or smaller than the first reference value, the processing diverts to step S19.

In step S18, the control unit 21 determines whether the predicted maximum value of the glucose concentration values in the interstitial fluid within the second period is equal to or larger than the second reference value. If the predicted maximum value of the glucose concentration values in the interstitial fluid within the second period is not equal to or larger than the second reference value, the processing loops back to step S11. For example, after an elapse of predetermined time, the control unit 21 executes the process in S11. If the predicted maximum value of the glucose concentration values in the interstitial fluid within the second period is equal to or larger than the second reference value, the processing diverts to step S19. In step S19, the control unit 21 selects insulin as the type of medicine, and controls the pump 33 to dose the medicine in vivo. The control unit 21 may select one of the plural dosage algorithms stored in the storage unit 22, based on the predicted glucose concentration value in the interstitial fluid within the second period. The control unit 21 may control the pump 33 to dose the medicine in vivo by using the selected dosage algorithm. In step S18, the control unit 21 may determine whether the predicted average value of the glucose concentration values in the interstitial fluid within the second period is equal to or larger than the second reference value. When the predicted average value of the glucose concentration values in the interstitial fluid within the second period is not equal to or larger than the second reference value, the processing loops back to step S11. Whereas when the predicted average value of the glucose concentration values in the interstitial fluid within the second period is equal to or larger than the second reference value, the processing proceeds to step S19.

According to the second embodiment, there is calculated the predicted glucose concentration value in the interstitial fluid within the second period ranging from the present time to the second time after the present time, corresponding to the user's activity status within the first period ranging from the first predetermined time before the present time to the present time. When the predicted glucose concentration value in the interstitial fluid within the second period does not fall within the range of the predetermined concentration value, the medicine is dosed in vivo. When the predicted minimum value or the predicted average value of the glucose concentration values in the interstitial fluid within the second period is equal to or smaller than the first reference value, glucagon is dosed in vivo, thereby enabling the glucose concentration value in the blood and the glucose concentration value in the interstitial fluid to be kept at the desired values. This contrivance reduces the risk that the user enters the hypoglycemic state. When the predicted maximum value or the predicted average value of the glucose concentration values in the interstitial fluid within the second period is equal to or larger than the second reference value, insulin is dosed in vivo, thereby enabling the glucose concentration value in the blood and the glucose concentration value in the interstitial fluid to be kept at the desired values. This contrivance reduces the risk that the user enters the hyperglycemic state. According to the second embodiment, it is therefore feasible to keep the glucose concentration value in the blood and the glucose concentration value in the interstitial fluid at the desired values by predicting the fluctuations of the glucose concentration value, which take into consideration the body motion status of the user and the body status pertaining to the life support (vital-signs) of the user.

According to the second embodiment, one of the plural dosage algorithms stored in the storage unit 22 is selected based on the predicted glucose concentration value in the interstitial fluid within the second period. The medicine is dosed in vivo by employing the selected dosage algorithm. It is therefore feasible to select the proper dosage algorithm by predicting the glucose concentration value in the interstitial fluid within the second period and to dose the medicine in vivo.

The first embodiment may be combined with the second embodiment. When the predicted glucose concentration value in the interstitial fluid within the second period does not fall within the range of the predetermined concentration value, the control unit 21 may execute the process of informing the user of the information about the glucose concentration value in the interstitial fluid, and the process of controlling the pump 33 to dose the medicine in vivo. When the predicted glucose concentration value in the interstitial fluid within the second period does not fall within the range of the predetermined concentration value, the control unit 21 may also execute at least one of the process of informing the user of the information about the glucose concentration value in the interstitial fluid, and the process of controlling the pump 33 to dose the medicine in vivo.

The control unit 34 of a dosage apparatus 3 may execute a part or a whole of the processes to be executed by the control unit 21 of the receiving apparatus 2. The control unit 34 may acquire the glucose concentration value in the interstitial fluid from the receiving apparatus 2. The control unit 34 may determine whether the predicted glucose concentration value in the interstitial fluid within the second period falls within the range of the predetermined concentration value. The control unit 34 may select glucagon or insulin as the type of medicine, and may control the pump 33 to dose the medicine in vivo. The storage unit 35 may store the plurality of dosage algorithms. The storage unit 35 is one example of a "second storage unit". The control unit 34 may select one of the plural dosage algorithms stored in the storage unit 35, based on the predicted glucose concentration value in the interstitial fluid within the second period. The control unit 34 may control the pump 33 to dose the medicine in vivo by using the selected dosage algorithm. The control unit 34 may also select one of the plural dosage algorithms stored in the storage unit 22, based on the predicted glucose concentration value in the interstitial fluid within the second period. The control unit 34 may acquire the selected dosage algorithm from the receiving apparatus 2 and may control the pump 33 to dose the medicine in vivo by using the selected dosage algorithm.

For example, a program (measuring program) may be stored in a memory of the computer equipped in at least one of the transmitting apparatus 1, the receiving apparatus 2 and the dosage apparatus 3, and may be run by the computer, whereby there respective processes in the first and second embodiments may also be attained. The computer may include a processor instanced by the CPU, a Micro Processing Unit (MPU) and a Field Programmable Gate Array (FPGA), and may also include a dedicated processor instanced by an Application Specific Integrated Circuit (ASIC). The respective processes in first and second embodiments may be attained based on a method (measuring method) by which the computer runs the program. The program may be provided to the computer via, e.g., a network or from a computer readable recording medium and other equivalent mediums that retain the data in a non-transitory manner. The program may be recorded on the computer readable recording medium.

The measurement system including the transmitting apparatus 1, the receiving apparatus 2 and the dosage apparatus 3 has been described so far, and the second embodiment is not, however, limited to this example. The transmitting apparatus 1, the receiving apparatus 2 and the dosage apparatus 3 may be provided integrally as the measurement apparatus. The transmitting apparatus 1 and the receiving apparatus 2 may be provided integrally as the measurement apparatus. The transmitting apparatus 1 and the dosage apparatus 3 may be provided integrally as the measurement apparatus. The receiving apparatus 2 and the dosage apparatus 3 may be provided integrally as the measurement apparatus. Any one of the transmitting apparatus 1 and the receiving apparatus 2 may also be provided as the measurement apparatus. The control unit 13 of the transmitting apparatus 1 may function as at least one of the "measurement unit", the "acquiring unit", the "calculation unit", and the "informing unit". The control unit 21 of the receiving apparatus 2 may function as at least one of the "measurement unit", the "acquiring unit", the "calculation unit", and the "informing unit". The control unit 34 of the dosage apparatus 3 may function as at least one of the "measurement unit", the "acquiring unit", the "calculation unit", and the "informing unit".

### <Computer Readable Recording Medium>

It is possible to record a program which causes a computer, machine, system (hereinafter, described as computer and other equivalent hardware components) to implement any of the functions described above on a computer readable recording medium. By causing the computer and other equivalent hardware components to read in the program from the recording medium and execute it, the function thereof can be provided. The computer readable recording medium mentioned herein indicates a recording medium which stores information such as data and a program by an electric, magnetic, optical, mechanical, or chemical operation and allows the stored information to be read from the computer and other equivalent hardware components. Of such recording media, those detachable from the computer or the like include, e.g., a flexible disk, a magneto-optical disk, a CD-ROM, a CD-R/W, a DVD, a Blu-ray disc, a DAT, an 8-mm tape, a flash memory and a memory card. Of such recording media, those fixed to the computer and other equivalent hardware components include a hard disk, a ROM or the like.

## Claims

1. A measurement apparatus comprising:
a measurement unit configured to measure a concentration value of a specified substance contained in a sample;
an acquiring unit configured to acquire information about a user's activity status;
a calculation unit configured to calculate a concentration value of the specified substance within a second period ranging from present time to second predetermined time after the present time, based on a concentration value of the specified substance within a first period ranging from first predetermined time before the present time to the present time and the information about the user's activity status within the first period; and
an informing unit configured to inform a user of the information about the concentration value of the specified substance when the concentration value of the specified substance within the second period does not fall within a range of a predetermined concentration value.

2. The measurement apparatus according to claim 1, further comprising a storage unit configured to store a plurality of calculation algorithms,
wherein the calculation unit selects one calculation algorithm from the storage unit, based on the information about the user's activity status within the first period, and calculates the concentration value of the specified substance within the second period from the concentration value of the specified substance within the first period by using the selected calculation algorithm.

3. The measurement apparatus according to claim 1 or 2, wherein the information about the user's activity status contains a user's activity quantity and user's biometric information.

4. The measurement apparatus according to any one of claims 1 through 3, wherein the specified substance is glucose.

5. The measurement apparatus according to claim 4, wherein the information about the concentration value of the specified substance contains information representing a hypoglycemic state and information representing a hyperglycemic state.

6. The measurement apparatus according to any one of claims 1 through 5, wherein the acquiring unit acquires the information about the user's activity status from at least one activity sensor, and
the at least one activity sensor includes a motion sensor configured to detect a motion quantity of the user.

7. The measurement apparatus according to any one of claims 1 through 6, wherein the acquiring unit acquires the information about the user's activity status from at least one activity sensor, and
the at least one activity sensor includes an attitude sensor configured to detect an attitude of the user.

8. The measurement apparatus according to any one of claims 1 through 7, wherein the acquiring unit acquires the information about the user's activity status from at least one activity sensor, and
the at least one activity sensor includes a temperature sensor configured to measure a body temperature of the user.

9. The measurement apparatus according to any one of claims 1 through 8, wherein the acquiring unit acquires the information about the user's activity status from at least one activity sensor, and
the at least one activity sensor includes a pulse sensor configured to measure a pulse rate of the user, a heart rate sensor configured to measure a heart rate of the user, or a pulse wave sensor configured to measure pulse waves of the user.

10. The measurement apparatus according to any one of claims 1 through 9, wherein the acquiring unit acquires the information about the user's activity status from at least one activity sensor, and
the at least one activity sensor includes a blood pressure sensor configured to measure a blood pressure value of the user.

11. The measurement apparatus according to any one of claims 1 through 10, wherein the measuring unit continuously measures the concentration values of the specified substance contained in the sample.

12. The measurement apparatus according to any one of claims 1 through 11, further comprising a control unit configured to control a dosing unit to dose a medicine in vivo,
wherein the control unit controls the dosing unit to dose the medicine in vivo when the concentration value of the specified substance within the second period does not fall within a range of a predetermined concentration value.

13. The measurement apparatus according to claim 12, further comprising a second storage unit configured to store a plurality of dosage algorithms,
wherein the control unit selects one dosage algorithm from the second storage unit, based on the concentration value of the specified substance within the second period and the information about the user's activity status, and controls the dosing unit to dose the medicine in vivo by using the selected dosage algorithm.

14. A measurement program for making a computer execute:
a process of measuring a concentration value of a specified substance contained in a sample;
a process of acquiring information about a user's activity status;
a process of calculating a concentration value of the specified substance within a second period ranging from present time to second predetermined time after the present time, based on a concentration value of the specified substance within a first period ranging from first predetermined time before the present time to the present time and the information about the user's activity status within the first period; and
a process of informing a user of the information about the concentration value of the specified substance when the concentration value of the specified substance within the second period does not fall within a range of a predetermined concentration value.

15. A measuring method comprising:
a process of measuring a concentration value of a specified substance contained in a sample;
a process of acquiring information about a user's activity status;
a process of calculating a concentration value of the specified substance within a second period ranging from present time to second predetermined time after the present time, based on a concentration value of the specified substance within a first period ranging from first predetermined time before the present time to the present time and the information about the user's activity status within the first period; and
a process of informing a user of the information about the concentration value of the specified substance when the concentration value of the specified substance within the second period does not fall within a range of a predetermined concentration value.
